# EUROPEAN PATENT SPECIFICATION

(11) **EP 0 514 603 B1**
(45) Date of publication and mention of the grant of the patent: **29.05.2002**
(21) Application number: 91308439.8
(22) Date of filing: 16.09.1991
(51) Int. Cl.: A61K 35/76, A61K 39/12, A61K 39/245, A61K 48/00, A61P 35/00

(54) **Viral mediated destruction of neoplastic cells**
Viral verursachte Zerstörung von Neoplastzellen
Destruction de cellules néoplastiques par médiateur viral

(30) Priority: 14.09.1990 US 582057
(43) Date of publication of application: 25.11.1992
(73) Proprietor: THE GENERAL HOSPITAL CORPORATION, Boston Massachusetts 02115 (US); THE PRESIDENT & FELLOWS OF HARVARD COLLEGE, Boston, Massachusetts 02115 (US)
(72) Inventor: Martuza, Robert L., Lexington, Massachusetts 01273 (US); Coen, Donald M., Medfield, Massachusetts 02052-2011 (US)
(74) Representative: Lee, Nicholas John

(56) References cited:
- WO-A-96/00007
- THE LANCET, vol. 1, June 6, 1970, London, H.E. WEBB et al. "Viruses in the treatment of cancer", pages 1206-1209
- PROCEEDINGS OF THE NATIONAL ACADEMY OF SCIENCES OF THE UNITED STATES OF AMERICA, vol. 86, no. 12, June 1989, Washington DC, D.M. COEN et al. "Thymidine Kinase-negative herpes simplex virus mutants establish latency in mouse trigeminal ganglia but do not reactivate.", pages 4736-4740
- ANNALS OF THE NEW YORK ACADEMY OF SCIENCES, vol. 54, art. 6, pages 865-1232, July 10, 1952, New York, A.E. MOORE "Viruses with oncolytic properties and their adaption to tumors.", pages 945-952
- Skinner G. R.B. et al. (1988) "Reduced tumorigenicity of rodent tumour cells and tumout explants following ingection with wild type and mutant herpes simplex virus, bovine mammillitis virus and encephalomyocarditis virus" Br. J. Exp. Path (69): 495-504
- Shimizu Y et al. (1988) "Immunotherapy of advanced gynecologic cancer patiens utilizing mumps virus" Cancer Detection and Prevention (12): 487-495
- Cowan M et al (1990) "Inhibition of rate of tumour growth in rodent species by inoculation of herpesviruses and encephalomyocarditis virus" Journal of Medical Virology (30): 211-215
- Lorence R M et al (1988) "Newcastle disease virus as an antineoplastic agent: induction of tumor necrosis factor-alpha and augmentation of its cytotoxicity" Journal of the National Cancer Institute (80): 1305-1312
- Markert J M et al (1993) "Reduction and elimination of encephalitis in an experimental glioma therapy model with attenuated herpes simplex mutants that retain susceptibility to acyclovir" Neurosurgery (32): 597-603
- Dubbs D R and Kit S (1964) "Mutant strains of herpes simplex deficient in thymidine kinase-inducing activity" Virology (22): 493-522

## Description

This invention relates to treatment of neoplastic cells using viruses.

Cancer is a disease of highly evolved multicellular organisms. The disease is best defined by the four characteristics which describe how cancer cells act differently from their normal counterparts. First, in most cases, cancer originates from a single cell which proliferates to form a clone of malignant cells. Second, cancer cells grow autonomously and are not properly regulated by the normal biochemical and physical influences in the cells environment. Third, cancer cells are anaplastic which is the lack of normal coordinated cell differentiation. Fourth, cancer cells develop the capacity for discontinuous growth and dissemination to other body parts which is called metastasis.

The terms cancer, neoplasia and malignancy usually are used interchangeably. The term cancer refers to the full spectrum of malignant neoplasms, of which there are over a hundred known types to affect humans (See Mendelsohn, J., in Harrison's Principles of Internal Medicine, ed. Braunwald et al., 1987, McGraw-Hill Inc., New York, p. 421-431). These can be broadly classified into three major types. Malignant neoplasms arising from epithelial structures are called carcinomas and constitute the vast majority of all malignant tumors. Malignant neoplasms that originate from connective tissues such as muscle, cartilage, fat or bone are called sarcomas, and malignant tumors affecting hematopoetic structures (structures pertaining to the formation of blood cells) including components of the immune system, are called leukemias and lymphomas. A tumor is the neoplastic growth of the disease cancer.

Neoplasia is a process by which the normal controlling mechanisms that regulate cell growth and differentiation are impaired resulting in progressive growth. During neoplasia, there is a characteristic failure to control cell turnover and growth. This lack of control causes a tumor to grow progressively, enlarging and occupying spaces in vital areas of the body. If the tumor invades surrounding tissue and is transported to distant sites, the tumor will likely cause death of the individual.

One-third of all individuals in the United States will develop cancer (American Cancer Society Yearly Outlook for 1990). The five year survival rate for these patients has risen to nearly 50% as a result of progress and early diagnosis and therapy of the disease (American Cancer Society Yearly Outlook for 1990). However, cancer remains second only to cardiac disease as a cause of death in this country (American Cancer Society Yearly Outlook for 1990). Nearly 20% of all Americans who die this year will die of cancer (American Cancer Society Yearly Outlook for 1990). Half of these deaths will be due to the three most common types of cancer: lung, breast, and colon.

Recently there has been a rapid expansion of cancer treatments. Even though new treatments are being developed, the need still exists for improved methods for the treatment of most types of cancers.

The preferential killing of cancer cells without deleterious effect on normal cells is the desired goal in cancer therapy. In the past this has been accomplished using a variety of procedures. These procedures include the administration of chemicals, chemotherapy, radiation, radiotherapy, and surgery.

Radiotherapy is a regional form of treatment used for the control of localized cancers (See Devita, V.T., in Harrison's Principles of Internal Medicine, ed. Braunwald et al., 1987, McGraw-Hill Inc., New York, p. 431-446). Radiotherapy relies on the fact that some malignant diseases are more susceptible to damage by radiation. This difference in susceptibility depends on normal cells having a higher capacity for intercellular repair than neoplastic cells and the ability of normal organs to continue to function well if they are only segmentally damaged. If surrounding tissue can tolerate twice the radiation dose of a given tumor, then the tumor is radiosensitive. On the other hand, some tumors cannot be treated with radiotherapy. Cancer which extensively involves both lungs cannot be treated effectively with radiation therapy because of the greater radiosensitivity of the surrounding lung tissue (See Devita, V.T., in Harrison's Principles of Internal Medicine, ed. Braunwald et al., 1987, McGraw-Hill Inc., New York, p. 431-446).

A more modern approach to the use of radiotherapy involves the use of chemicals as radiosensitizers. Chemicals such as n-ethylmaleimide or a synthesis blocker like buthionine sulfoximine can render cells radio sensitive and hence more susceptible to killing by radiation. These chemicals are in the early phases of development are not yet commercially available (See Devita, V.T., in Harrison's Principles of Internal Medicine, ed. Braunwald et al., 1987, McGraw-Hill Inc., New York, p. 431-446).

Surgery is still considered the primary treatment for most early cancers (See Devita, V.T., in Harrison's Principles of Internal Medicine, ed. Braunwald et al., 1987, McGraw-Hill Inc., New York, p. 431-446). However, most tumors are operable but not fully resectable. Some tumors that appear resectable have micrometastatic disease outside the tumor field. This leads to a recurrence of the cancer close to the initial site of occurrence. Any cancer showing a level of metatastis effectively cannot be cured through surgery.

Other types of localized therapy (nonsystemic) have been explored. These include local hypothermia (Salcman et al., J. Neuro-Oncol. 1:225-236 (1983)), photodynamic therapy (Cheng et al., Surg. Neurol. 25:423-435 (1986)), and interstitial radiation (Gutin et al., J. Neurosurgery 67:864-873 (1987)). To date these therapies have been met with limited success.

Radiotherapy and surgery offers ways of reducing the tumor mass in specific regions of the body that are accessible through surgical techniques or high doses of radiotherapy. Neither is applicable to the destruction of widely disseminated or circulating tumor cells characteristically present in most patients with cancer. This is the stimulus of the development of systemic treatments of cancer such as chemotherapy.

Chemotherapy involves the administration of toxic compounds systemically to a patient (Chabner, B.A., ed., Pharmacologic Principles of Cancer Treatment, Philadelphia, Saunders (1982)). Since cancer cells are growing more rapidly than normal cells, toxic compounds are more cytotoxic to cells undergoing rapid division.

Drug development for cancer began with the accidental identification of the lymphocytic activity of mustard gas used in World War I and II. Nitrogen mustard, an antitumor drug, is a derivative of mustard gas and was used to treatment lymphomas in the 1940's (See Devita, V.T., in Harrison's Principles of Internal Medicine, ed. Braunwald et al., 1987, McGraw-Hill Inc., New York, p. 431-446). Most of the early patients treated with nitrogen mustard had subsequent relapses. This was followed by an overwhelming disappointment and skepticism that cancer could be successfully treated with drugs.

The next drug investigated for antitumor activity was methotrexate (See Devita, V.T., in Harrison's Principles of Internal Medicine, ed. Braunwald et al., 1987, McGraw-Hill Inc., New York, p. 431-446). Methotrexate, an antimetabolite, was first used successfully against acute childhood leukemia. In some of the early cases where methotrexate was used, remission produced by the drug appeared permanent.

New compounds are constantly being generated and selected both by rational drug design and random screening. There are six major classes of antitumor drugs; alkylating agents, antimetabolites, plant alkaloids, antitumor antibiotics, endocrine agents, as well as some miscellaneous drugs (Myers, C.E., in Cancer: Principles and Practice of Oncology, 2d ed., V.T. De Vito et al. (eds.), Philadelphia, Lippincott, 1985, pp. 290-328).

The use of chemicals, even though widespread in use, has proved of limited effectiveness in treating most cancer types. A major limitation of current chemotherapy is that it is effective only against the most rapidly dividing tumor cells. An additional drawback to the use of cytotoxic agents for the treatment of cancer are their severe side effects. These include nausea, vomiting, CNS depression, localized pain, bone marrow depression, bleeding, renal damage, hypo and hyperglycemia and hypersensitivity reactions.

A more modern approach to chemotherapy is to direct the toxic agents to the cancer cells themselves. This has been accomplished experimentally by linking the chemotherapeutic agent to either antibodies or nontoxic molecules that have a higher affinity for the tumor cells than normal cells. These directed toxic therapies are still in an early clinical phase of development and are not commercially available.

Certain types of cancer, for example gliomas, which are the most common primary tumour arising in the human brain, defy the current modalities of treatment. Despite surgery, chemotherapy, and radiotherapy, glio-blastoma, the most common of the gliomas is almost universally fatal (Schoenberg, B.S., "The epidemiology of nervous system tumors," in Oncology of the Nervous System, M.D. Walker, ed., Boston, MA, Martinus Nijhoff (1983); Levin et al., "Neoplasms of the Central Nervous System," Chapter 46, pp. 1557-1611, in Cancer: Principles and Practice of Oncology, vol. 2, 3rd ed., De Vita et al., eds., Philadelphia, Lippincott Press (1989)). Therefore a need exists for the development of a technique that will selectively destroy glioma while sparing normal brain cells. In general, such treatment could potentially be used universally for the selective destruction of all types of neoplastic cells.

Thus according to a first aspect of the present invention there is provided the use of a herpes virus HSV-1, which has a mutated thymidine kinase gene such that it is genetically-altered so as to be capable of replication in neoplastic cells but substantially unable to replicate in non-neoplastic cells, for the preparation of a pharmaceutical composition for treating nervous system tumours in humans.

The virus can be suitably mutated to prevent spontaneous reversion to the wild type virus for example, by deleting one or more portions of nucleic acid.

In some embodiments the invention also extends to hosts that are transfected (or transformed) with the virus.

The neoplastic cells suitably comprise cells of nervous system tumours. Such tumours include astrocytoma, oligodenroglioma, meningioma, neuro- fibroma, ependymoma, Schwannoma and neurofibrosarcoma. In particular, glioblastoma tumours are of interest.

The virus may be HSV-1-dlsptk.

The virus may be altered to express tumour suppressor genes in preferred embodiments.

The virus may comprise one or more heterologous promoters which can be used to express proteins necessary for viral replication. Suitably such heterologous promoters are selectively capable of expression in neoplastic cells.

Thus the neoplastic cells may be killed (selectively) by the virus either by oncolysis and/or xenogenization.

Particularly preferred embodiments of the invention relate to the use of a virus described in accordance with the first aspect for selectively killing or destroying glioblastoma cells.

Upon viral infection, the virus destroys infected cells, generally by oncolysis and/or xenogenization.

The viral infection leads to destruction of the neoplastic cells without causing systemic viral infection.

As indicated, the viruses selectively kill neoplastic cells and the malignant and benign neoplastic cells. By "neoplastic cells" is intended rapidly dividing cells. For purposes of the invention, neoplastic cells include cells of tumors, neoplasms, carcinomas, sarcomas, leukemias, lymphomas, and the like. Of particular interest are nervous system tumors. These include astrocytomas, oligo-dendrogliomas, meningiomas, neurofibromas, ependymomas, Schwannomas, neurofibrosarcomas, glioblastomas, etc.

Gliomas are the most common primary tumors arising in the human brain. The most malignant glioma, the glioblastoma, represents approximately 30% to 50% of all primary brain tumors and, despite surgery, chemotherapy, and radiotherapy, are almost universally fatal (Moore, A.E., Progr. Exp. Tumor Res. 1;411-439 (1960)). The mean survival is less than a year, and the five-year survival rate is only 3% to 5%. After treatment, reoccurrence of the disease often appears within 2 centimeters of the original site (Hochberg et al., Neurol. 30:907-911 (1980)). Metastases are extremely rare; neurological dysfunction and death are due to local growth and cerebral invasion. Therefore, the possible efficacy of local (non-systemic) treatments has been explored. A few of these include studies of local hypothermia (Saloman et al., J. Neuro-Oncol. 1:225-236 (1983)), photodynamic therapy (Cheng et al., Surg. Neurol. 25:423-435 (1986)), and interstitial radiation (Gutin et al., J. Neurosurgery 67:864-873 (1987)). To date, no therapeutic modality has made a substantial impact on the outcome of patients with malignant gliomas.

The virus may be altered by mutagenesis as well as genetic engineering techniques.

In general, viruses are defined as infectious units comprising either RNA or DNA enclosed in a protective coat (See Hull, R. et al., in Virology: Directory and Dictionary of Animal, Bacterial and Plant Viruses, New York, Stockton Press, 1989). The nucleic acid contains information necessary for the replication of the virus in a susceptible host cell. Viruses contain no energy producing enzyme systems, no functional ribosomes, and no cellular organelles. These are supplied by the cell in which the viruses are replicated. The cell may also supply some of the enzymes necessary for viral replication, such as DNA or RNA polymerase and other replication factors.

Methods for mutagenesis are well known in the art. These include a variety of techniques, including chemical mutagenesis (Chu, C.T. et al., Virology 98:168 (1979); Myers, R.M. et al., Science 229:242 (1985)), oligonucleotide-mediated mutagenesis (Zoller, M.J. et al., DNA 3:479 (1984)), and the like.

Alternatively, the virus can be altered by insertion of foreign nucleic acids. In this manner, proteins or factors necessary for viral replication are placed under the control of heterologous promoters and/or heterologous control elements such that the protein is expressed in neoplastic cells only. The heterologous promoters and control elements for the most part are expressed selectively or at a higher level in neoplastic cells. For example, the promoter of a neoplastic gene which is expressed only in neoplastic cells may be utilized. Alternatively, promoters from genes which are expressed at higher levels in rapidly dividing cells, such as thymidine kinase, polymerase genes, etc., may be utilized.

Methods for the construction of engineered viruses are known in the art. Generally these include Sambrook et al., Molecular Cloning: A Laboratory Manual, 2nd Ed., Cold Spring Harbor Laboratory Press (1989) and the references cited therein. Virological considerations are also reviewed in Coen D.M, Molecular Genetics of Animal Viruses in Virology, 2^{nd} Edition, B.N. Fields (editor), Raven Press, NY (1990) pp. 123-150, and the references cited therein. References drawn specifically to herpes simplex virus 1 (HSV-1) include: Geller et al., Science 241:1667 (1988); Geller et al., Proc. Natl. Acad. Sci. USA 87:1149 (1990); Geller, A.I., Nucl. Acid Res. 16:5690 (1988); Breakfield et al., Molec. Neurobiol. 1:339 (1987); Shih et al., in: Vaccines 85, Cold Spring Harbor Press, Cold Spring Harbor, NY (1985), pp. 177-180; Palella et al., Molec. Cell. Biol. 8:457 (1988); Matz et al., J. Gen. Virol. 64:2261 (1983);; Smile J.R., Nature 285:333 (1980); Mocarski et al. Cell 22:243, (1980); Coen et al., Science 234:53 (1986).

As taught by the references, when using heterologous promoters, the gene of interest is placed in the proper reading frame with respect to the heterologous promoter. In this manner, the heterologous promoter directs the transcription of the gene.

Treatment with viruses as discussed below has not progressed because of the drawbacks of using unaltered or wild type viruses.

In the past, wild type viruses have been explored as a treatment for the tumors in both animals and in humans (Moore, A.E., Progr. Exp. Tumor Res. 1:411-439 (1960); Austin, F.C. et al., Ad. in Cancer Res. 30:301-345 (1979)). The proposed therapeutic mechanisms include oncolysis, direct cell killing by the virus (Moore, A.E., Progr. Exp. Tumor Res. 1:411-439 (1960)), and xenogenization the production of new antigens on the tumor cell surface to facilitate an immunological rejection of the tumor (Austin, F.C. et al., Ad. in Cancer Res. 30:301-345 (1979); Kobayashi, H. et al., Ad. in Cancer Res. 30:279-299 (1979)).

There are several early references to the beneficial effects of viral infection on the progression of malignant diseases (Levanditi, Ann. Inst. Pasteur 37:1-106 (1923)). Regressive changes have been noted in various tumor types when the patients have either been deliberately or accidentally infected with a virus. In 1912 regression was observed in a patient with cervical carcinoma undergoing rabies treatment for a dog bite. It was then postulated that a rabies virus may have been the cause of tumor regression (Southom, C.M., Tran. N.Y. Acad. Sci. Ser. 2122:653-673 (1960)).

Between 1950 and 1967 there were numerous reports that suggested some patients showed regressive changes in their cancers when treated with viruses (Moore, A.E., Ann. Rev. Microbiol. 8:393-410 (1954); Moore, A.E., Progr. Exp. Tumor Res. 1:411-439 (1960)). In 1956, the use of viruses to treat carcinoma of the cervix was reported (Smith et al., Cancer 9:1211-1218 (1956)). Use of the adeno virus resulted in the slowing of the tumor growth after oncolysis. However, no appreciable modification in the course of the disease was noted. In 1965, the use of Newcastle disease virus for the treatment of cervical carcinoma was attempted (Cassel, W.A. et al., Cancer 18:863-868 (1965)). NDV was chosen because it has a very low degree of neurotropism, the ability to infect neural tissues. Direct inoculation of the virus into the carcinoma of the cervix resulted in extensive sloughing of the tumor and the shrinkage of the lymphnode metastasis. The use of NDV was deemed hopeful due to the fact that this patient showed no evidence of a virus attacking the nerve tissues. In 1966, a report of a clinical trial using viruses to treat other cancer was published (Webb, H.E. et al., Lancet 1:1206-1209 (1966). The viruses used in this study were Lungat and Kyasanur Forest disease virus.

More recent studies have been published concerning the use of viruses to treat cancers. In 1983, a study was published on the use of the Newcastle disease virus oncolysates in the management of stage II to malignant melanoma (Cassel, W.A. et al., Cancer 52:856-860 (1983)). Patients received subcutaneous injections of viral oncolysates over a three year period. Even though these patients were at an advanced stage of disease, stage II, the results suggested an improvement. The mortality rate of 6, 8 and 12 percent in each of the 3 years respectively in the study group was considerably lower than that in the control group. Viral replication is not noted in the study and the effects noted were probably due to the xenogenization of the entire tumor cells.

Roenigle et al. (Roenigle, H.H. et al., Arch. Dermatol. 109:668-673 (1974)) report on the positive result of immunotherapy of malignant melanoma with interlesional inoculations of vaccinia virus. Major regression of the tumors were observed in 8 out of 8 patients with stage II disease. In other studies with patients in stage III disease, little or no regression was observed. Additionally, work utilizing vaccinia virus therapy demonstrating positive result have been published (Belisario, J.C. et al., Aust. J. Derm. 6:113-118 (1961); Milton, G.W. et al., Aust. N.Z. J. Surg. 35:286-290 (1966); Hunter-Craig, I. et al., Br. Med. J. 2:512-515 (1970)). These have been performed primarily in patients without extensive tumor involvement.

Several animal models and animal tumors have also been utilized in the study of oncolysis by viruses in tissue culture and in whole animal systems (Moore, A.E., Ann. Rev. Microbiol. 8:393-410 (1954); Moore, A.E., Progr. Exp. Tumor Res. 1:411-439 (1960)). At least nine viruses have been shown to be capable of causing tumor regression to some degree in a variety of tumors in mice, rats, rabbits, and guinea pigs. However, a major drawback of these early animal studies was systemic infection by the virus used to treat the tumors.

In contrast to prior studies, the present invention utilizes altered viruses. These altered viruses, for the most part, are not capable of replication in certain normal cells, thus reducing the threat of systemic infection. It will be demonstrated that viruses can be altered to selectively replicate in and kill neoplastic cells, specifically by utilizing a mutant HSV-1 to kill glioma cells, the method can be utilized to alter other viruses such as those discussed above.

The use of viruses in tumor therapy is also beneficial as the virus modifies the tumor cells so that they become more antigenic. See, for example, Araki et al. Gene 89:195-202 (1990); Takle et al. Mol. Biochem. Parasitol 37:57-64 (1989); and Burk et al. J. Virol. 62:649-654 (1988). This antigenic effect is accomplished by introducing new antigens on the surface of the tumor thus augmenting one's immune system in recognizing the tumor as a foreign body. The introduction of new antigens to the surface of the tumor is referred to as xenogenization of tumors (Austin, F.C. et al., Ad. in Cancer Res. 30:301-345 (1979); Kobayashi, H. et al., Ad. in Cancer Res. 30:279-299 (1979)).

When tumors cells are infected with a virus, they generally express the viral antigen on the cell surface. When the virus used to infect the tumor cells are recognized as foreign to the hosts, then the virus infected tumor cells often undergo regression. Kobayashi (Kobayashi, H. et al., J. Natl. Cancer Inst. 42:413-419 (1969)) attempted to promote the tumor rejection in rats with tumors by establishing a viral infection of the host with murine leukemia viruses such as Friend virus or Gross virus. These viruses were believed to produce surfacing antigens on host cells. Success was noted in the immunological regression of these rat tumors. Several other reports have been noted with viral induced regression of transplanted tumors in rats (Holtermann, D.A. et al., Transplantation 11:20-29 (1971); Barbieri, D., et al. Int. J. Cancer 7:364-1971 (1971); Greenberger, J.S. et al., J. Natl. Cancer Inst. 51:1935-1938 (1973)).

Successful tumor regression in systems other rats have been noted. In 1971, it was reported that the lethal growth of mouse tumors was decreased by 46 to 77 percent when the tumor cells were infected with Rauscher leukemia virus (Barbieri, D. et al., Int. J. Cancer 7:364-371 (1971)). Holterman and Majde Holtermann, D.A. et al., Transplantation 11:20-29 (1971) reported that the lethal growth of a adeno carcinoma in SWR/J mice was decreased 50 percent if the tumor cells were first infected with the LCM virus.

Another study also reported the decrease in the lethal growth of tumors following the infection with the HVJ virus was noted in the Hamster model system (Yamada, T. et al., Gann. 63:647-655 (1972)). However, viral growth in the host limited the effectiveness and continuation of this potential therapy.

Virus may also be used to insert new genes into tumor cells which can alter cell growth characteristics or modulate cell growth. An example of this is the insertion of a tumor suppressor gene as has been shown for the Rb gene in retinoblastoma or osteo gene sarcoma (Huang H-JS et al., Suppression of the neoplastic phenotype by replacement if the Rb gene in human cancer cells in Science 242: 1563-1566 (1988)) or the p53 gene in colon cancer (Baker **et al.,** Suppression of human colorectal carcinoma cell growth by wild-type p53 in Science 246:912-915 1980)). In addition, other suppressor or modulating genes may be used.

Attempts have been made to modify viruses naturally to make them more capable of infecting tumor cells (Moore, A.E., Ann. N.Y. Acad. Sci. 54:945-952 (1952); Southam, C. et al., Virology 5:395-400 (1958); Cassel, W.A., Can. Res. 17:618-622 (1957)). This has been accomplished through serial passages of the viruses through tumor lines in vitro. In most cases where an increase in tumor specificity has been noted a reversion back to the wild type viral infection spectrum was noted after serial passage of virus back into the host organism. It would be more desirable to control specificity in a more reliable fashion than to randomly select for specificity through passaging.

The present viruses, altered by genetic engineering techniques or mutation, are targeted to specific cell types. This targeting may be able to be performed by a variety of techniques.

Tumor specific control elements can be introduced into the virus genome to control the expression of any one or more of the viral genes necessary for viral replication. In doing so the virus would only be able to replicate in cells in which the tumor specific control element was expressed. This would eliminate the potentiality of inducing a systemic viral infection in the host when these viruses are used for cancer treatment.

As an alternative to tumor specific control elements, specific mutations can be introduced into the virus genome such that the virus would be unable to replicate in cells unless the cell provided a complementary enzymatic functions. This would lead to a directed infection of the tumor cells only.

The virus can be tested in vitro for the ability to replicate and suppress or destroy growth of neoplastic cells. The virus is thus altered by mutation or genetic engineering techniques such that the virus will replicate and cause oncolysis and/or xenogenization and/or cell growth modulation in tumor cells while being unable to replicate in normal cells. The virus is then used to establish localized infection within neoplastic cells of a patient.

To effect viral infection, the virus is typically injected into the host at or near the site of neoplastic growth although systemic inoculation may also be feasible if cell-specific viruses are used. Generally, the virus is provided for injection in a concentration in the range of about 10¹ to about 10¹⁰ plaque forming units (PFU) generally about 5x10⁴ PFUs to about 1x10⁶ PFU, more generally about 1x10⁵ PFU to about 4x10⁵ PFU although ranges may vary.

The pharmaceutical composition may be suitable for injection (i.e., an injectable composition) in which case it is preferably sterile.

The pharmaceutical composition may be prepared by a process comprising admixing an altered virus which is capable of replication in neoplastic cells but not in normal cells with a pharmaceutically acceptable carrier.

The following Example is offered for illustration and is not to be construed as limiting the present invention.

### EXAMPLE

Glioblastomas are the most common form of malignant brain tumours in man, and are almost always universally fatal (Schoenberg, B.S., "The epidemiology of nervous system tumors," in Oncology of the Nervous System, M.D. Walker, ed., Boston, MA, Martinus Nijhoff (1983); Levin et al., "Neoplasms of the Central Nervous System," Chapter 46, pp. 1557-1611, in Cancer: Principles and Practice of Oncology, vol. 2, 3rd ed., De Vita et al., eds., Philadelphia, Lippincott Press (1989)). Human malignant glioma cells are a class of rapidly dividing tumour cell population which express enzymes associated with DNA replication. One of these is the enzyme thymidine kinase. The surrounding normal brain is mostly composed of non-dividing cells, such as neurons and normal gila. These cells express enzymes involved in DNA replication at a minimal level. The present invention is directed to take advantage of this phenomenon. A virus, such as Herpes simplex virus 1 is mutated in one of the viral genes, such as the enzyme thymidine kinase. This mutant virus is used to treat the tumor. This is accomplished by the virus's ability to replicate within the malignant glioma cells using the tumor cell's endogenous thymidine kinase activity, to complement the mutation. This leads to the lysis of the tumor cell. In contrast, normal brain cells, which are non-dividing are relatively non-permissive for lytic infection by some mutant viruses and are spared from viral lysis. (Jamieson AT, J. Gen. Virol. 24:465 (1974).

The possibility of using a mutant virus to treat human cancer was explored using the virus dlsptk (Coen et al., Proc. Natl. Acad. Sci. USA 86:4736 (1989)). This particular mutant was chosen among several possible thymidine kinase mutants of HSV1 because it has no detectable thymidine kinase activity and because it has a large deleted segment. This large deletion makes this virus unable to spontaneously revert to wild-type virus. A third advantage is that the deletion lies outside the HSV UL24 gene, which overlaps the thymidine kinase gene and thus dlsptk should not be selective for UL24 function. Reported herein are the results of testing dlsptk virus versus five different human gliomas: two in long-term culture and three in short-term culture. Also is reported the results of inoculating dlsptk intercranially into rodents and of the intraneoplastic inoculation of human gliomas grown in two different anatomical locations in nude mice with dlsptk.

The human glioma lines U87 and T98G cells were obtained from the American Tissue Cell Collection (ATCC), Camden, NJ. Three primary human malignant glioma cultures were started from explants obtained from surgical specimens at the Massachusetts General Hospital. Vero (African green monkey kidney) cells were obtained from the ATCC. All cell cultures were grown on plastic tissue culture dishes in 5% CO₂ at 37°C using Delbeco's modified Eagle's medium supplemented with 10% fetal bovine serum and antibiotics (DME).

Viruses were grown and tittered on Vero cells as previously described and are recorded as plaque forming units (PFU) per specified volume. Susceptibility to foscarnet and vidarabine were assayed by plague reduction to demonstrate that dlsptk retained sensitivity to these drugs which have been used in patients to treat serious HSV infection.

All animal studies were done in accordance with guidelines for animal care as defined by both the National Institute of Health and by the Massachusetts General Hospital Subcommittee on Animal Care. Rats (CDF Fisher male; 150-175 grams) were obtained from Charles River; nude mice were obtained from the breeding colonies at the Massachusetts General Hospital. Each were housed in facilities appropriate for their care. All culture and animal procedures involving viruses were approved by the Harvard Environmental Safety Committee.

KOS is a standard laboratory wild type strain. HSV1-dlsptk was constructed as described by Coen et al., Proc. Natl. Acad. Sci. USA 86:4736 (1989). In the example discussed, the sensitivity of the glioma cell lines were tested for infection for KOS and dlsptk. In addition, the effects of intracranial inoculation of KOS and dlsptk was assayed. Finally, the effects of dlsptk on tumors implanted in three different positions of a body of mice was assayed.

In cell culture, either wild type virus KOS or the thymidine kinase negative mutant (dlsptk) were applied to multiplicities of infection (MOI) ranging from 10⁻⁴ to 10¹ to U87 cells and to Vero cells. With both cell types, the number of cells showing cytopathic effect at 24 hours was directly related to the multiplicity of infection and ranged from 1-5% at MOI=10⁻⁴ to 95% at MOI=10. At 24 hours after viral application, the extent of cytopathic effect and cell detachment in the U87 cells was equal to or greater than that seen in the Vero cells at equal MOI. By five days following infection, cytopathic effect was 100% for both viruses in both cell types at all MOI≥10⁻¹; by nine days 100% cytopathic effect was evident even for the plate inoculated with dlsptk at an MOI=10⁻⁴. This suggested that even at a very low inoculum (an MOI of 10⁻⁴ is equivalent to 10 pfu/culture plate), dlsptk was able to sustain a spreading infection and to destroy the entire monolayer of U87 cells in 9 days.

In order to determine if dlsptk would lyse a different human glioma line in cell culture, T98G and Vero cells were inoculated with dlsptk at an MOI=10. Both viruses produced complete cell destruction of each line within several days.

Since both U87 and T98G are long term lines of human gliomas, the effect of HSV1-dlsptk was determined on primary human gliomas. Monolayer cell cultures were derived by explanation from three primary glioma specimens obtained at surgery and studied at the second passage. Following application of dlsptk at MOI=10 or MOI=1, all 3 primary gliomas demonstrated cytopathic effect in a dose dependent fashion. By four days, 100% cell destruction was evident in all 3 cultures at both MOI tested.

In order to establish the safety of intracerebral inoculation of dlsptk, 13 male Fischer rats (150-175g) stereotactically inoculated in the right frontal lobe with 2x10⁵ pfu HSV-dlsptk in 2 µl DME and 12 rats with 1x10⁶ HSV-dlsptk in 4.8 µl DME. Two of the 2x10⁵ pfu group died at the time of anesthesia and surgery. All remaining animals were followed for at least one month following inoculation. There were no deaths or neurologic dysfunction in either group. The 2x10⁵ pfu group were allowed to live an additional month before sacrifice. At two months, all were healthy and showed no evidence of neurologic dysfunction nor cataracts. This is in contrast to prior studies by Chiocca et al. showing that a similar dose of the wild type virus (KOS, 2x10 pfu) killed 718 rats within two weeks after intracranial inoculation.

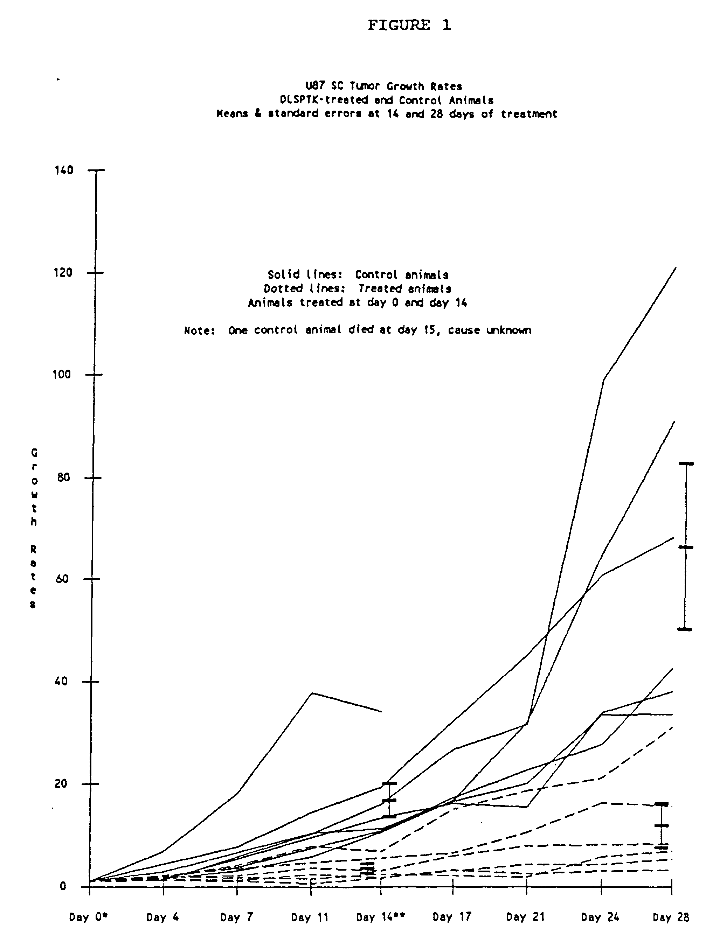

To test the effects of HSV-dlsptk on U87 tumors in vivo, a group of 17 nude mice were injected subcutaneously in the left buttock area with 3.2x10⁶ U87 cells. Growing tumors were evident at approximately 5 weeks. 12 animals had tumors with diameters of greater than 8 mm. These were randomly divided into two groups. Using a bevel tip Hamilton syringe, one group of 6 animals received an intraneoplastic inoculation of 5x10⁶ pfu HSV1-dlsptk in 25µl DME. The control group of 7 received a similar inoculation of DME alone. After two weeks the tumors were reinjected with twice the dose. The tumors receiving injections of dlsptk were significantly smaller than the controls after 2 and 4 weeks of measurement (Figure 1).

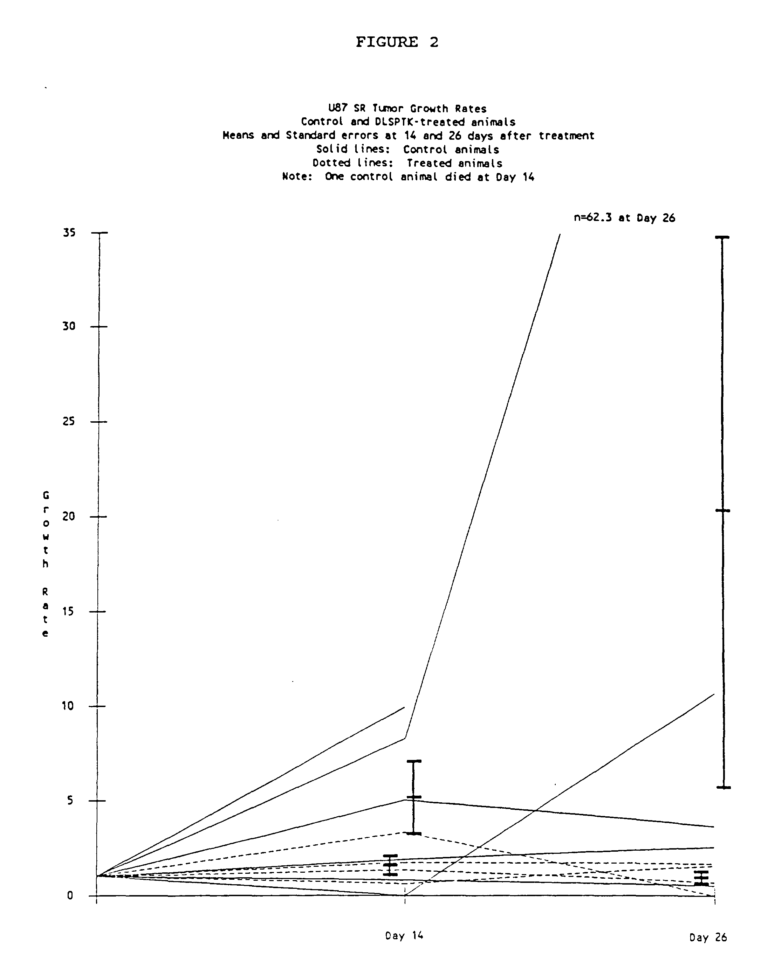

The effectiveness of dlsptk was also tested at a second location. Nine nude mice were inoculated in the subrenal capsule with U87 cells using the fibrin clot technique previously described. After 2 weeks, tumour sizes were measured with an ocular micrometer and found to be 1 to 2 mm in diameter. Half the animals were inoculated intraneoplastically with 1 µl DME and the other half were inoculated intraneoplastically with 1 µl DME containing 2.1X10⁵ pfu HSV-dlsptk. Figure 2 shows that at reexploration at day 14 and day 26 after inoculation, the dlsptk treated tumours were significantly smaller than the controls.

## Claims

1. The use of a herpes virus HSV-1, which has a mutated thymidine kinase gene such that it is genetically-altered so as to be capable of replication in neoplastic cells but substantially unable to replicate in non-neoplastic cells, for the preparation of a pharmaceutical composition for treating nervous system tumours in humans.

2. The use according to claim 1, wherein the tumour is an astrocytoma, a oligodendroglioma, a meningioma, a neurofibroma, an ependymoma, a Schwannoma, a neurofibrosarcoma and/or a glioblastoma.

3. The use according to claim 1 wherein the virus is HSV-1-dlsptk.

4. The use according to any one of claims 1 to 3, wherein the virus comprises one or more heterologous promoters which are used to express proteins necessary for viral replication and/or the virus expresses tumour suppressor genes.

## Patentansprüche

1. Verwendung eines Herpesvirus HSV-1, welches ein mutiertes Thymidinkinase-Gen hat, sodaß es genetisch verändert ist, um fähig zur Replikation in neoplastischen Zellen, aber im wesentlichen unfähig zur Replikation in nicht-neoplastischen Zellen zu sein, zur Zubereitung einer pharmazeutischen Zusammensetzung zur Behandlung von Tumoren des Nervensystems in Menschen.

2. Verwendung nach Anspruch 1, wobei der Tumor ein Astrozytom, ein Oligodendrogliom, ein Meningeom, ein Neurofibrom, ein Ependymom, ein Schwannom, ein Neurofibrosarkom und/oder ein Glioblastom ist.

3. Verwendung nach Anspruch 1, wobei das Virus ein HSV-1-dlsptk ist.

4. Verwendung nach einem der Ansprüche 1 bis 3, wobei das Virus ein oder mehrere heterologe Promotoren aufweist, welche daran gewöhnt sind, Proteine, die zur viralen Replikation notwendig sind, zu exprimieren, und/oder das Virus Tumorsuppressorgene exprimiert.

## Revendications

1. Utilisation d'un virus herpétique HSV-1 qui a un gène thymidine kinase muté tel qu'il est génétiquement modifié de façon à être capable d'une réplication en cellules néoplasiques mais essentiellement incapable de répliquer en cellules non néoplasiques, pour la préparation d'une composition pharmaceutique pour le traitement de tumeurs du système nerveux chez les humains.

2. Utilisation selon la revendication 1, dans laquelle la tumeur est un astrocytome, un oligodendrogliome, un méningiome, un neurofibrome, un épendymome, un Schwannome, un neurofibrosarcome et/ou un glioblastome.

3. Utilisation selon la revendication 1, dans laquelle le virus est le HSV-1-dlsptk.

4. Utilisation selon l'une quelconque des revendications 1 à 3, dans laquelle le virus comprend un ou plusieurs promoteurs hétérologues qui sont utilisés pour exprimer des protéines nécessaires pour une réplication virale et/ou le virus exprime des gènes suppresseurs de tumeur.
